# EUROPÄISCHE PATENTSCHRIFT

(11) **EP 0 450 345 B1**
(45) Veröffentlichungstag und Bekanntmachung des Hinweises auf die Patenterteilung: **24.05.1995**
(21) Anmeldenummer: 91103628.3
(22) Anmeldetag: 09.03.1991
(51) Int. Cl.: C07D 453/02, A61K 31/435

(54) **Indolderivate**
Indole derivatives
Dérivés d'indole

(30) Priorität: 24.03.1990 DE 4009565
(43) Veröffentlichungstag der Anmeldung: 09.10.1991
(73) Patentinhaber: MERCK PATENT GmbH, D-64271 Darmstadt (DE)
(72) Erfinder: Böttcher, Henning, Dr., W-6100 Darmstadt (DE); Seyfried, Christoph, Dr., W-6104 Jugenheim (DE); Minck, Klaus-Otto, Dr., W-6105 Ober-Ramstadt (DE); Wolf, Hans-Peter, Prof. Dr., W-6146 Alsbach (DE)

(56) Entgegenhaltungen:
- EP-A- 0 190 920
- EP-A- 0 323 077
- EP-A- 0 361 629
- WO-A-90/14347
- DE-A- 3 810 552
- NATURE, Band 316, 11. Juli 1985, Seiten 126-131; B.P. RICHARDSON et al.: "Identification of serotonin M-receptor subtypes and their specific blockade by a new class of drugs"
- JOURNAL OF ORGANIC CHEMISTRY, Band 33, Nr. 2, Februar 1968, Seiten 487-490; R.J. SUNDBERG: "Indoles from o-Nitrostyrenes. Synthesis and reactions of 2-Indolyl 4-Piperidylmethyl Ketone"
- JOURNAL OF ORGANIC CHEMISTRY, Band 38, Nr. 17, 24. August 1973, Seiten 3004-30011; R.L. AUGUSTINE et al.: "Synthesis of alpha-Monosubstituted Indoles"
- JOURNAL OF ORGANIC CHEMISTRY, Band 40, Nr. 17, 22. August 1975, Seiten 2525-2529; K. FRETER: "3-Cycloalkenylindoles"

## Beschreibung

Gegenstand der Erfindung sind neue 3-(3-Indolyl)-chinuclidin-Derivate der Formel I

Ind - R I

worin
- Ind: eine unsubstituierte oder eine ein- bis vierfach durch 1-4-C-Alkyl, Trifluormethyl, 1-4-C-Alkoxy, 1-4-C-Alkylthio, Fluor, Chlor, Hydroxy, Hydroxymethyl, 6-10-C-Aryloxy, 7-11-C-Aralkyloxy, 1-5-C-Acyloxy, 6-10-C-Aroyloxy, 1-4-C-Alkylsulfonyloxy, 6-10-C-Arylsulfonyloxy, Carboxy, 1-4-C-Alkoxycarbonyl und/oder Methylendioxy substituierte 3-Indolylgruppe und
- R: 3-Chinuclidinyl oder 2,3-Dehydro-3-chinuclidinyl bedeuten,
und deren Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. Diese Wirkungen erlauben es, diese Substanzen zur Behandlung von Erkrankungen, die durch einen Überschuß an zirkulierendem Serotonin oder durch eine serotonerge Überfunktion charakterisiert sind, einzusetzen. Dazu gehören insbesondere die Behandlung von Psychosen, von Nausea und Erbrechen (wie sie z.B. bei der chemo- oder radiotherapeutischen Behandlung von Krebserkrankungen auftreten), von Dementia oder anderen kognitiven Erkrankungen, von Migräne und von Suchterkrankungen. Dazu gehören weiterhin die Anwendung als Anxiolytikum, als Antiaggressivum, als Antidepressivum und als Analgetikum. Im einzelnen antagonisieren die Verbindungen die Wirkung von Serotonin an 5-HT₃-Rezeptoren, wie z.B. den durch Serotonin hervorgerufenen von-Bezold-Jarisch-Reflex (Methodik siehe J.Pharm.Pharmacol. 40 (1980), 301-302 und Nature 316 (1985), 126-131). Außerdem verdrängen die neuen Verbindungen die als selektiver 5-HT₃-Ligand bekannte Substanz ³H-GR65630 von homogenisiertem Gewebe aus dem endorhinalen Cortex der Ratte (siehe Europ.J.Pharmacol. 159 (1989), 157-164).

Die Verbindungen I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Ähnliche Verbindungen sind in J.Org.Chem. 40, 2525-2529 (1975), in J.Org. Chem. 38, 3004-3011 (1973) und in J.Org.Chem. 33, 487-490 (1968) beschrieben. In allen diesen Fällen sind jedoch keine pharmakologischen Wirkungen angegeben.

DE-A-3 810 552 beschreibt Ester und Amide von Indolcarbonsäuren.

Im Rest Ind bedeutet 1-4-C-Alkyl vorzugsweise Methyl, ferner auch Ethyl, n-Propyl, Isopropyl, n-Butyl, Isobutyl, sek.-Butyl oder tert.-Butyl. 1-4-C-Alkoxy ist vorzugsweise Methoxy, ferner auch Ethoxy, n-Propoxy, Isopropoxy, n-Butoxy, Isobutoxy, sek.-Butoxy oder tert.-Butoxy, 1-5-C-Acyloxy vorzugsweise Formyloxy oder Acetyloxy, Propanoyloxy, n-Butanoyloxy, Isobutanoyloxy oder Pivaloyloxy. 1-4-C-Alkylsulfonyloxy ist vorzugsweise Methansulfonyloxy. 6-10-C-Aryloxy ist bevorzugt Phenyloxy, 7-11-C-Aralkyloxy ist bevorzugt Benzyloxy, 6-10-C-Aroyloxy ist bevorzugt Benzoyloxy.

Bevorzugt bedeutet der Rest Ind eine unsubstituierte oder einfach oder zweifach substituierte 3-Indolylgruppe. Falls Ind eine substituierte 3-Indolylgruppe bedeutet, so ist sie vorzugsweise in 2-, 5- und/oder 6-Stellung substituiert und/oder in 1-Stellung alkyliert. Im einzelnen bedeutet Ind vorzugsweise 3-Indolyl, 5-Benzyloxy-3-indolyl, 5-Hydroxy-3-indolyl und 5-Methoxy-2-methyl-3-indolyl. Der Rest R ist bevorzugt 3-Chinuclidinyl.

Gegenstand der Erfindung ist ferner ein Verfahren zur Herstellung von Verbindungen der Formel I sowie von ihren Salzen, dadurch gekennzeichnet, daß man Verbindungen der Formel II,

Ind-H II

wobei Ind die angegebene Bedeutung hat,
mit 3-Chinuclidinon oder einem seiner Salze zur 2,3-Dehydro-3-chinuclidinylverbindung (I,R = 2,3-Dehydro-3-chinuclidinyl) umsetzt und diese soweit gewünscht zur 3-Chinuclidinylverbindung (I, R = 3-Chinuclidinyl) reduziert, und/oder eine Verbindung, die sonst der Formel I entspricht, aber an Stelle eines oder mehrerer H-Atome eine abspaltbare Schutzgruppe enthält, durch Abspaltung dieser Schutzgruppe in eine Verbindung der Formel I überführt und/oder in einer Verbindung der Formel I einen Rest Ind in einen anderen Rest Ind umwandelt und/oder eine Base der Formel I durch Behandeln mit einer Säure in eines ihrer Salze umwandelt und/oder aus einem Salz einer Base der Formel I diese mittels einer starken Base freisetzt.

Die Herstellung der Verbindung der Formel I erfolgt im übrigen nach an sich bekannten Methoden, wie sie in der Literatur (z.B. J. March, Advanced Organic Chemistry, 3rd edition, John Wiley & Sons, New York oder Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu einer Verbindung der Formel I umsetzt.

Die Herstellung einer Verbindung der Formel I (R = 2,3-Dehydro-3-chinuclidinyl) erfolgt vorzugsweise, indem man eine Verbindung der Formel II und 3-Chinuclidinon oder eines seiner Salze entweder in einem basischen Medium oder in einem sauren Medium bei 40 bis 120 °C umsetzt. Die Reaktionszeit beträgt bevorzugt 2 bis 60 Stunden.

Für das alkalische Medium wird zweckmäßigerweise ein inertes, mit Wasser mischbares organisches Lösungsmittel zusammen mit wäßriger KOH-Lösung benutzt; besonders bevorzugte organischen Lösungsmittel sind niedrige Alkanole, wie Methanol oder Ethanol.

Für die Umsetzung im sauren Medium dient vorzugsweise eine Mischung aus einer mit Wasser mischbaren organischen Säure und einer wäßrigen Lösung einer anorganischen Säure; insbesonders bevorzugte organische Säuren sind niedere Carbonsäuren, wie Essigsäure; Phosphorsäure wird als anorganische Säure bevorzugt.

Die erhaltene Verbindung der Formel I (R = 2,3-Dehydro-3-chinuclidinyl) kann anschließend falls erwünscht zu der entsprechenden Verbindung der Formel I (R = 3-Chinuclidinyl) reduziert werden. Für die Reduktion sind katalytische Hydrierungen geeignet, vorzugsweise an einem Edelmetallkatalysator wie Platinoxid oder Palladium auf Kohle in einem inerten Lösungsmittel wie Methanol, Ethanol, Tetrahydrofuran (THF) oder Ethylacetat, bei Temperaturen zwischen 0 und 150 °C vorzugsweise zwischen 10 und 80 °C, und Drucken zwischen 1 und 200, vorzugsweise 1 und 10 bar.

Die Hydrierung der Doppelbindung kann auch durch Diboran in THF erfolgen. Das Diboran kann in situ aus Natriumborhydrid und Bortrifluorid-Ether-Komplex hergestellt werden.

Die Verbindungen der Formel I (R = 3-Chinuclidinyl) enthalten zumindestens ein asymmetrisches Kohlenstoffatom. Sie können daher als Racemate, falls mehrere asymmetrische Kohlenstoffatome vorhanden sind, auch als Gemische mehrerer Racemate sowie in verschiedenen optisch-aktiven Formen vorliegen.

Die Verbindungen der Formel II sind zum Teil bekannt; die nicht bekannten Verbindungen können leicht analog zu den bekannten Verbindungen hergestellt werden. So können z.B. N- oder O-acylierte Verbindungen aus den nicht-acylierten Vorstufen durch Umsetzung mit Säureanhydriden, z.B. Acetanhydrid, in basischen organischen Lösungsmitteln, z.B. Pyridin, hergestellt werden.

Man kann weiterhin aus Verbindungen, die sonst der Formel I entsprechen, aber an Stelle eines oder mehrerer H-Atome eine abspaltbare Schutzgruppe, insbesondere eine hydrogenolytisch abspaltbare Schutzgruppe, wie Benzyloxy, enthalten, diese mittels katalytischer Hydrierung abspalten, wobei man Verbindungen der Formel I erhält. Weiterhin können im besonderen Verbindungen, die sonst der Formel I entsprechen, aber an Stelle eines oder mehrerer H-Atome eine solvolytisch abspaltbare Schutzgruppe, wie Acyl (z.B. Acetyl) oder Sulfonyl (z.B. Methansulfonyl oder Toluolsulfonyl), enthalten, zu Verbindungen der Formel I solvolysiert, insbesondere hydrolysiert werden.

Die Ausgangsstoffe für die Solvolyse sind beispielsweise erhältlich durch Reaktionen von 1-Z-Ind-H mit 3-Chinuclidinon oder einem seiner Salze, wobei Ind die angegebene Bedeutung besitzt und Z eine solvolytisch abspaltbare Gruppe ist. So können insbesondere Verbindungen der Formel I, wobei der Rest Ind in der 1-Stellung des Indols eine Acylgruppe, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl, enthält, zu den entsprechenden in 1-Stellung unsubstituierten Verbindungen hydrolysiert werden, z.B. in saurem, besser neutralem oder alkalischem Medium bei Temperaturen zwischen 0 und 200 °C. Als Basen verwendet man zweckmäßig Natrium-, Kalium- oder Calciumhydroxid, Natrium- oder Kaliumcarbonat oder Ammoniak. Als Lösungsmittel wählt man vorzugsweise Wasser, niedere Alkohole wie Methanol oder Ethanol, Ether wie THF oder Dioxan, Sulfone wie Tetramethylensulfon oder deren Gemische, besonders die Wasser enthaltenden Gemische. Eine Hydrolyse kann bereits beim Behandeln mit Wasser allein erfolgen, insbesondere in der Siedehitze.

Man kann in einer Verbindung der Formel I einen Rest Ind in einen anderen Rest Ind umwandeln, indem man z.B. eine Ethergruppe spaltet, wobei das entsprechende Hydroxyderivat entsteht, und/oder eine Carboxylgruppe verestert und/oder eine Estergruppe verseift und/oder eine Carboxylgruppe durch Decarboxylierung abspaltet. So kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250 °C, oder durch Behandeln mit Diisobutylaluminiumhydrid in Toluol bei etwa 0-110 °C, oder durch katalytische Hydrierung, z.B. in Gegenwart von Palladium-Kohle in einem der oben genannten inerten Lösungsmitteln, z.B. Methanol, bei z.B. 0 bis 50 °C und bei z.B. 1 bis 10 bar. Die genannten Veresterungen erfolgen z.B. durch die Behandlung einer Lösung der Carbonsäure mit einem Alkohol unter Zusatz von SOCl₂ oder eines wasserabspaltenden Mittels, wobei ein Überschuß des Alkohols vorzugsweise als Lösungsmittel dient. Die Hydrolyse von Carbonsäureestern erfolgt z.B. mittels saurer oder basischer Katalyse in einer wäßrigen Lösung, die zusätzlich ein inertes mit Wasser mischbares organisches Lösungsmittel, wie Dioxan, enthalten kann. Decarboxylierungen werden zweckmäßigerweise in alkalischem Medium, z.B. in N,N-Dimethylanilin bei Temperaturen zwischen 40 und 190 °C, vorzugsweise zwischen 160 und 190 °C, ausgeführt.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- und Ethansulfonsäure, Benzolsulfonsäure, p-Toluolsulfonsäure, Napthalin-mono- und -disulfonsäuren, Laurylschwefelsäure. Säureadditionssalze, die nicht physiologisch unbedenklich sind (z.B. Pikrate), können sich zur Isolierung und Aufreinigung der Basen der Formel I eignen.

Eine Base der Formel I kann, falls erwünscht, aus einem ihrer Salze mit starken Basen wie Natrium- oder Kaliumhydroxid, Natrium- oder Kaliumcarbonat in Freiheit gesetzt werden.

Gegenstand der Erfindung ist ferner die Verwendung von Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(n) in eine geeignete Darreichungsform gebracht werden.

Gegenstand der Erfindung sind ferner Mittel, insbesondere pharmazeutische Zubereitungen, enthaltend eine oder mehrere Verbindungen der Formel I und/oder ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendungen Salben, Cremes, Pflaster oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht auch einen oder mehrere weitere Wirkstoffe enthalten, z.B. ein oder mehrere Vitamine.

Verbindungen der Formel I und ihre physiologisch unbedenklichen Salze können bei der therapeutischen Behandlung des menschlichen oder tierischen Körpers und bei der Bekämpfung von Krankheiten verwendet werden, insbesondere von Krankheiten, die mit einem Überschuß an zirkulierendem Serotonin oder durch eine serotonerge Überfunktion gekennzeichnet sind. Zu diesen Krankheiten gehören insbesondere Psychosen, Nausea und Erbrechen, wie sie als Begleitsymptome bei der chemo- und radiotherapeutischen Behandlung von Tumoren entstehen, Dementia und andere kognitive Erkrankungen, Migräne und Suchterkrankungen. Weiterhin gehören anxiolytische, analgetische und anti-aggressive Wirkung in diesen Anwendungsbereich.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0.2 und 1000 mg, insbesondere zwischen 0.2 und 100 mg pro Dosierungseinheit. Die tägliche Dosierung liegt vorzugsweise zwischen etwa 0.003 und 20 mg/kg Körpergewicht. Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Anwendung ist bevorzugt.

### Beispiele

In den nachstehenden Beispielen bedeutet "übliche Aufarbeitung": Man gibt, falls erforderlich, Wasser hinzu, extrahiert mit Ethylacetat oder Ether, trennt ab, trocknet die organische Phase über Natriumsulfat, filtriert, dampft ein und reinigt durch Chromatographie an Kieselgel und/oder durch Kristallisation. Temperaturen sind in Grad Celsius angegeben. Rf bedeutet, wenn nicht anders angegeben, Rf-Wert auf Kieselgel mit dem Laufmittel Toluol/Methanol/Triethylamin (7:2:1).

### Beispiel 1

Zu einer Lösung von 2,3 g Indol und 3,2 g 3-Chinuclidinon in 120 ml Methanol gibt man eine Lösung von 2,2 g KOH in 20 ml Wasser; diese Mischung wird 16 Stunden gekocht. Das Methanol wird abdestilliert und der ölige Rückstand mit Wasser und Ether versetzt, dabei kristallisiert 3-(3-Indolyl)-2,3-dehydrochinuclidin, F. 250° (Zersetzung); Rf 0,28.

Analog werden folgende Verbindungen erhalten:
3-(4-Methoxy-3-indolyl)-2,3-dehydro-chinuclidin
3-(5-Methoxy-3-indolyl)-2,3-dehydro-chinuclidin, F. 223-224°
3-(5-Ethoxy-3-indolyl)-2,3-dehydro-chinuclidin
3-(5-Propoxy-3-indolyl)-2,3-dehydro-chinuclidin
3-(6-Methoxy-3-indolyl)-2,3-dehydro-chinuclidin
3-(5,6-Dimethoxy-3-indolyl)-2,3-dehydro-chinuclidin
3-(5,6-Methylendioxy-3-indolyl)-2,3-dehydro-chinuclidin, F. 280-281°
3-(4,5,6-Trimethoxy-3-indolyl)-2,3-dehydro-chinuclidin
3-(5-Methylthio-3-indolyl)-2,3-dehydro-chinuclidin
3-(5-Fluor-3-indolyl)-2,3-dehydro-chinuclidin
3-(5-Chlor-3-indolyl)-2,3-dehydro-chinuclidin
3-(5-Trifluormethyl-3-indolyl)-2,3-dehydro-chinuclidin
3-(6-Chlor-5-methoxy-3-indolyl)-2,3-dehydro-chinuclidin
3-(6-Methyl-3-indolyl)-2,3-dehydro-chinuclidin
3-(6-Methoxy-1-methyl-3-indolyl)-2,3-dehydro-chinuclidin
3-(6-Ethoxy-1-methyl-3-indolyl)-2,3-dehydro-chinuclidin
3-(6-Propoxy-1-methyl-3-indolyl)-2,3-dehydro-chinuclidin

### Beispiel 2

3,2 g 5-Methoxy-2-methylindol werden in 60 ml Essigsäure gelöst und auf 70° erhitzt; anschließend werden 4,8 g 3-Chinuclidinonhydrochlorid und 14,5 ml 2 N Phosphorsäure zugegeben. Die Reaktionslösung wird 20 Stunden bei 65° gehalten, dann auf Eiswasser gegossen und mit NaOH-Lösung alkalisch gestellt. Der erhaltene Niederschlag wird abgesaugt und mit Wasser gewaschen. Nach Extraktion (Ether) und Chromatographie wird das Produkt in Ether gelöst und mit isopropanolischer Salzsäure angesäuert, wobei das 3-(5-Methoxy-2-methyl-3-indolyl)-2,3-dehydro-chinuclidin als Hydrochlorid ausfällt; F. 240,9 - 241,5° (Zersetzung); Rf 0,28.

Analog werden folgende Verbindungen erhalten:
3-(2-Methyl-3-indolyl)-2,3-dehydro-chinuclidin; Rf 0,20
3-(5-Ethoxy-2-methyl-3-indolyl)-2,3-dehydro-chinuclidin
3-(5-Propoxy-2-methyl-3-indolyl)-2,3-dehydro-chinuclidin
3-(6-Methoxy-2-methyl-3-indolyl)-2,3-dehydro-chinuclidin
3-(5,6-Dimethoxy-2-methyl-3-indolyl)-2,3-dehydro-chinuclidin
3-(5,6-Methylendioxy-2-methyl-3-indolyl)-2,3-dehydro-chinuclidin
3-(4,5,6-Trimethoxy-2-methyl-3-indolyl)-2,3-dehydro-chinuclidin
3-(5-Methylthio-2-methyl-3-indolyl)-2,3-dehydro-chinuclidin
3-(5-Fluor-2-methyl-3-indolyl)-2,3-dehydro-chinuclidin
3-(5-Chlor-2-methyl-3-indolyl)-2,3-dehydro-chinuclidin
3-(5-Trifluormethyl-2-methyl-3-indolyl)-2,3-dehydro-chinuclidin
3-(6-Chlor-5-methoxy-2-methyl-3-indolyl)-2,3-dehydro-chinuclidin
3-(2,6-Dimethyl-3-indolyl)-2,3-dehydro-chinuclidin
3-(5-Methoxy-1,2-dimethyl-3-indolyl)-2,3-dehydro-chinuclidin, F. 140-143°
3-(6-Ethoxy-1,2-dimethyl-3-indolyl)-2,3-dehydro-chinuclidin
3-(6-Propoxy-1,2-dimethyl-3-indolyl)-2,3-dehydro-chinuclidin

### Beispiel 3

0,4 g 3-(3-Indolyl)-2,3-dehydro-chinuclidin werden in 30 ml Methanol gelöst und mit 0,2 g Palladium-Kohle (5 %) bei 26° hydriert, bis keine weitere Gasaufnahme stattfindet. Die Hydrierlösung wird eingeengt und der ölige Rückstand aus Toluol kristallisiert. Es entsteht 3-(3-Indolyl)-chinuclidin, F. 213,8 - 215,8°; Rf 0,02.

Analog werden folgende Verbindungen erhalten:
3-(4-Methoxy-3-indolyl)-chinuclidin
3-(5-Methoxy-3-indolyl)-chinuclidin, F. 193-195°
3-(5-Ethoxy-3-indolyl)-chinuclidin
3-(5-Propoxy-3-indolyl)-chinuclidin
3-(6-Methoxy-3-indolyl)-chinuclidin
3-(5,6-Dimethoxy-3-indolyl)-chinuclidin
3-(5,6-Methylendioxy-3-indolyl)-chinuclidin, F. 243-245°
3-(4,5,6-Trimethoxy-3-indolyl)-chinuclidin
3-(5-Methylthio-3-indolyl)-chinuclidin
3-(5-Fluor-3-indolyl)-chinuclidin
3-(5-Chlor-3-indolyl)-chinuclidin
3-(5-Trifluormethyl-3-indolyl)-chinuclidin
3-(6-Chlor-5-methoxy-3-indolyl)-chinuclidin
3-(6-Methyl-3-indolyl)-chinuclidin
3-(6-Methoxy-1-methyl-3-indolyl)-chinuclidin
3-(6-Ethoxy-1-methyl-3-indolyl)-chinuclidin
3-(6-Propoxy-1-methyl-3-indolyl)-chinuclidin
3-(2-Methyl-3-indolyl)-chinuclidin, F. 206,8 - 208,6°
3-(5-Methoxy-2-methyl-3-indolyl)-chinuclidin Hydrochlorid, F. 231,7 - 234,0°
3-(5-Ethoxy-2-methyl-3-indolyl)-chinuclidin
3-(5-Propoxy-2-methyl-3-indolyl)-chinuclidin
3-(6-Methoxy-2-methyl-3-indolyl)-chinuclidin
3-(5,6-Dimethoxy-2-methyl-3-indolyl)-chinuclidin
3-(5,6-Methylendioxy-2-methyl-3-indolyl)-chinuclidin
3-(4,5,6-Trimethoxy-2-methyl-3-indolyl)-chinuclidin
3-(5-Methylthio-2-methyl-3-indolyl)-chinuclidin
3-(5-Fluor-2-methyl-3-indolyl)-chinuclidin
3-(5-Chlor-2-methyl-3-indolyl)-chinuclidin
3-(5-Trifluormethyl-2-methyl-3-indolyl)-chinuclidin
3-(6-Chlor-5-methoxy-2-methyl-3-indolyl)-chinuclidin
3-(2,6-Dimethyl-3-indolyl)-chinuclidin
3-(5-Methoxy-1,2-dimethyl-3-indolyl)-chinuclidin, Hydrochlorid, F. 237-238°
3-(6-Ethoxy-1,2-dimethyl-3-indolyl)-chinuclidin
3-(6-Propoxy-1,2-dimethyl-3-indolyl)-chinuclidin

### Beispiel 4

4,5 g 5-Benzyloxyindol und 3,2 g 3-Chinuclidinonhydrochlorid werden in 120 ml Methanol gelöst, und eine Lösung von 2,2 g KOH in 20 ml Wasser wird hinzugefügt. Die Mischung wird 44 Stunden gekocht. Das Methanol wird abdestilliert, der Rückstand wie üblich aufgearbeitet. Man erhält 3-(5-Benzyloxy-3-indolyl)-2,3-dehydrochinuclidin, F. 209,8 - 210,5°; Rf 0,18.

Analog werden folgende Verbindungen erhalten:
3-(6-Benzyloxy-3-indolyl)-2,3-dehydro-chinuclidin
3-(6-Benzyloxy-1-methyl-3-indolyl)-2,3-dehydro-chinuclidin
3-(5-Benzyloxy-3-indolyl)-2,3-dehydro-chinuclidin
3-(5-Benzyloxy-1-methyl-3-indolyl)-2,3-dehydro-chinuclidin
3-(6-Benzyloxy-2-methyl-3-indolyl)-2,3-dehydro-chinuclidin
3-(5-Benzyloxy-2-methyl-3-indolyl)-2,3-dehydro-chinuclidin
3-(6-Benzyloxy-1,2-dimethyl-3-indolyl)-2,3-dehydro-chinuclidin
3-(5-Benzyloxy-1,2-dimethyl-3-indolyl)-2,3-dehydro-chinuclidin.

### Beispiel 5

0,7 g 3-(5-Benzyloxy-3-indolyl)-2,3-dehydro-chinuclidin werden in 50 ml Methanol gelöst und mit 1,0 g Palladium-Kohle (5 %) bei 21° hydriert, bis keine weitere Gasaufnahme stattfindet. Die Hydrierlösung wird eingeengt und der Rückstand in Wasser gelöst und mit NaOH neutralisiert. Das ausgefallene 3-(5-Hydroxy-3-indolyl)-chinuclidin wird chromatographisch gereinigt, F. 280° (Zersetzung); Rf 0,2 (Methanol/Triethylamin; 8:2).

Analog werden folgende Verbindungen erhalten:
3-(6-Hydroxy-3-indolyl)-chinuclidin
3-(6-Hydroxy-1-methyl-3-indolyl)-chinuclidin
3-(5-Hydroxy-3-indolyl)-chinuclidin
3-(5-Hydroxy-1-methyl-3-indolyl)-chinuclidin
3-(6-Hydroxy-2-methyl-3-indolyl)-chinuclidin
3-(5-Hydroxy-2-methyl-3-indolyl)-chinuclidin
3-(6-Hydroxy-1,2-dimethyl-3-indolyl)-chinuclidin
3-(5-Hydroxy-1,2-dimethyl-3-indolyl)-chinuclidin

### Beispiel 6

200 mg 3-(5-Hydroxy-3-indolyl)-chinuclidin werden in 10 ml Pyridin gelöst und 5 ml Acetanhydrid unter Kühlung zugegeben. Nach einer Stunde wird wie üblich aufgearbeitet. Man erhält 3-(5-Acetoxy-3-indolyl)-chinuclidin.

Analog werden folgende Verbindungen erhalten:
3-(5-Pivaloyloxy-3-indolyl)-chinuclidin
3-(5-Propanoyloxy-3-indolyl)-chinuclidin
3-(5-Butanoyloxy-3-indolyl)-chinuclidin
3-(5-Methansulfonyloxy-3-indolyl)-chinuclidin
3-(6-Acetoxy-3-indolyl)-chinuclidin
3-(6-Pivaloyloxy-3-indolyl)-chinuclidin
3-(6-Propanoyloxy-indolyl)-chinuclidin
3-(6-Butanoyloxy-3-indolyl)-chinuclidin
3-(6-Methansulfonyloxy-3-indolyl)-chinuclidin
3-(5-Acetoxy-2-methyl-3-indolyl)-chinuclidin
3-(5-Pivaloyloxy-2-methyl-3-indolyl)-chinuclidin
3-(5-Propanoyloxy-2-methyl-3-indolyl)-chinuclidin
3-(5-Butanoyloxy-2-methyl-3-indolyl)-chinuclidin
3-(5-Methansulfonyloxy-2-methyl-3-indolyl)-chinuclidin
3-(6-Acetoxy-2-methyl-3-indolyl)-chinuclidin
3-(6-Pivaloyloxy-2-methyl-3-indolyl)-chinuclidin
3-(6-Propanoyloxy-2-methyl-3-indolyl)-chinuclidin
3-(6-Butanoyloxy-2-methyl-3-indolyl)-chinuclidin
3-(6-Methansulfonyloxy-2-methyl-3-indolyl)-chinuclidin

### Beispiel 7

200 mg 3-(1-Acetyl-5-methoxy-2-methyl-3-indolyl)-chinuclidin werden mit 100 mg KOH in 7 ml Wasser und 14 ml Ethanol 16 Stunden auf dem Dampfbad gerührt. Anschließend wird wie üblich aufgearbeitet. Man erhält 3-(5-Methoxy-2-methyl-3-indolyl)-chinuclidin; Hydrochlorid, F. 231,7 - 234,0°.

### Beispiel 8

100 mg 3-(3-Chinuclidinyl)-5-methoxyindol-2-carbonsäure werden in N,N-Dimethylanilin 8 Stunden bei 180° gerührt. Anschließend wird wie üblich aufgearbeitet. Man erhält 3-(5-Methoxy-3-indolyl)-chinuclidin.

### Beispiel 9

Analog Beispiel 1 erhält man mit 6-Hydroxymethyl-indol das 3-(6-Hydroxymethyl-3-indolyl)-2,3-dehydro-chinuclidin, F. > 399°.

Analog erhält man mit 4- bzw. 5-Hydroxymethyl-indol:
3-(4-Hydroxymethyl-3-indolyl)-2,3-dehydro-chinuclidin
3-(5-Hydroxymethyl-3-indolyl)-2,3-dehydro-chinuclidin.

### Beispiel 10

Analog Beispiel 3 erhält man durch Hydrierung der entsprechenden 2,3-Dehydrochinuclidine:
3-(4-Hydroxymethyl-3-indolyl)-chinuclidin
3-(5-Hydroxymethyl-3-indolyl)-chinuclidin
3-(6-Hydroxymethyl-3-indolyl)-chinuclidin, F. 248-250°.

Die nachstehenden Beispiele betreffen pharmazeutische Zubereitungen, die Substanzen der Formel I oder eines ihrer Säureadditionssalze enthalten:

### Beispiel A: Tabletten

Ein Gemisch von 1 kg 3-(5-Hydroxy-3-indolyl)-chinuclidinhydrochlorid, 4 kg Lactose, 1,2 kg Kartoffelstärke, 0,2 kg Talk und 0,1 kg Magnesiumstearat wird in üblicher Weise zu Tabletten verpreßt, so daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

2 kg 3-(5-Hydroxy-3-indolyl)-chinuclidin-hydrochlorid werden in üblicher Weise in Hartgelatinekapseln gefüllt, so daß jede Kapsel 20 mg Wirkstoff enthält.

### Beispiel D: Ampullen

Eine Lösung von 1 kg 3-(5-Hydroxy-3-indolyl)-chinuclidinhydrochlorid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die eine andere Verbindung der Formel I und/oder ein oder mehrere physiologisch unbedenkliche Säureadditionssalze einer Verbindung der Formel I enthalten.

## Patentansprüche (Patentansprüche für folgende(n) Vertragsstaat(en): AT, BE, CH, LI, DE, DK, FR, GB, GR, IT, LU, NL, SE)

1. 3-(3-Indolyl)-chinuclidine der Formel I
Ind - R I
worin
Ind eine unsubstituierte oder eine ein- bis vierfach durch 1-4-C-Alkyl, Trifluormethyl, 1-4-C-Alkoxy, 1-4-C-Alkylthio, Fluor, Chlor, Hydroxy, Hydroxymethyl, 6-10-C-Aryloxy, 7-11C-Aralkyloxy,, 1-5-C-Acyloxy, 6-10-C-Aroyloxy, 1-4-C-Alkylsulfonyloxy, 6-10-C-Arylsulfonyloxy, Carboxy, 1-4-C-Alkoxycarbonyl und/oder Methylendioxy substituierte 3-Indolylgruppe
und
R 3-Chinuclidinyl oder 2,3-Dehydro-3-chinuclidinyl
bedeuten,
sowie deren Salze.

2. Verfahren zur Herstellung von Verbindungen der Formel I sowie von deren Salzen, dadurch gekennzeichnet, daß man Verbindungen der Formel II,
Ind-H II
wobei der Rest Ind die in Anspruch 1 gegebene Bedeutung besitzt, mit 3-Chinuclidinon oder einem seiner Salze zur 2,3-Dehydro-3-chinuclidinylverbindung (I, R = 2,3-Dehydro-3-chinuclidinyl) umsetzt und diese soweit gewünscht zur 3-Chinuclidinylverbindung (I, R = 3-Chinuclidinyl) reduziert, und/oder eine Verbindung, die sonst der Formel I entspricht, aber an Stelle eines oder mehrerer H-Atome eine abspaltbare Schutzgruppe, enthält, durch Abspaltung dieser Schutzgruppe in eine Verbindung der Formel I überführt und/oder in einer Verbindung der Formel I einen Rest Ind in einen anderen Rest Ind und/oder eine Base der Formel I durch Behandeln mit einer Säure in eines ihrer Salze umwandelt.

3. Verfahren zur Herstellung einer pharmazeutischen Zubereitung, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Darreichungsform bringt.

4. Pharmazeutische Zubereitung, gekennzeichnet durch einen Gehalt an einer Verbindung der Formel I und/oder einem ihrer physiologisch unbedenklichen Salze.

5. Verwendung einer Verbindung der Formel I und/oder eines ihrer physiologisch unbedenklichen Salze zur Herstellung von Arzneimitteln.

## Patentansprüche (Patentansprüche für folgende(n) Vertragsstaat(en): ES)

1. Verfahren zur Herstellung von 3-(3-Indolyl)-chinuclidin-Derivaten der Formel I
Ind - R I
worin
Ind eine unsubstituierte oder eine ein- bis vierfach durch 1-4-C-Alkyl, Trifluormethyl, 1-4-C-Alkoxy, 1-4-C-Alkylthio, Fluor, Chlor, Hydroxy, Hydroxymethyl, 6-10-C-Aryloxy, 7-11C-Aralkyloxy,, 1-5-C-Acyloxy, 6-10-C-Aroyloxy, 1-4-C-Alkylsulfonyloxy, 6-10-C-Arylsulfonyloxy, Carboxy, 1-4-C-Alkoxycarbonyl und/oder Methylendioxy substituierte 3-Indolylgruppe
und
R 3-Chinuclidinyl oder 2,3-Dehydro-3-chinuclidinyl
bedeuten,
sowie von deren Salzen, dadurch gekennzeichnet, daß man Verbindungen der Formel II,
Ind-H II
mit 3-Chinuclidinon oder einem seiner Salze zur 2,3-Dehydro-3-chinuclidinylverbindung (I, R = 2,3-Dehydro-3-chinuclidinyl) umsetzt und diese soweit gewünscht zur 3-Chinuclidinylverbindung (I, R = 3-Chinuclidinyl) reduziert, und/oder eine Verbindung, die sonst der Formel I entspricht, aber an Stelle eines oder mehrerer H-Atome eine abspaltbare Schutzgruppe, enthält, durch Abspaltung dieser Schutzgruppe in eine Verbindung der Formel I überführt und/oder in einer Verbindung der Formel I einen Rest Ind in einen anderen Rest Ind und/oder eine Base der Formel I durch Behandeln mit einer Säure in eines ihrer Salze umwandelt.

2. Verfahren zur Herstellung einer pharmazeutischen Zubereitung, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Darreichungsform bringt.

## Claims (Claims for the following Contracting State(s): AT, BE, CH, LI, DE, DK, FR, GB, GR, IT, LU, NL, SE)

1. 3-(3-Indolyl)quinuclidines of the formula I
Ind - R I
in which
Ind is a 3-indolyl group which is unsubstituted or monosubstituted to tetrasubstituted by 1-4-C-alkyl, trifluoromethyl, 1-4-C-alkoxy, 1-4-C-alkylthio, fluorine, chlorine, hydroxyl, hydroxymethyl, 6-10-C-aryloxy, 7-11-C-aralkyloxy, 1-5-C-acyloxy, 6-10-C-aroyloxy, 1-4-C-alkylsulfonyloxy, 6-10-C-arylsulfonyloxy, carboxyl, 1-4-C-alkoxycarbonyl and/or methylenedioxy and
R is 3-quinuclidinyl or 2,3-dehydro-3-quinuclidinyl, and their salts.

2. Process for the preparation of compounds of the formula I and of their salts, characterised in that compounds of the formula II
Ind-H II
where the radical Ind has the meaning given in Claim 1, are reacted with 3-quinuclidinone or one of its salts to give the 2,3-dehydro-3-quinuclidinyl compound (I,R = 2,3-dehydro-3-quinuclidinyl) and this is reduced, if desired, to the 3-quinuclidinyl compound (I,R = 3-quinuclidinyl), and/or a compound which otherwise corresponds to the formula I, but contains a removable protecting group instead of one or more H atoms, is converted into a compound of the formula I by removing this protecting group and/or a radical Ind in a compound of the formula I is converted into another radical Ind and/or a base of the formula I is converted into one of its salts by treating with an acid.

3. Process for the production of a pharmaceutical preparation, characterised in that a compound of the formula I and/or one of its physiologically acceptable salts is brought into a suitable administration form together with at least one solid, liquid or semi-liquid excipient or auxiliary.

4. Pharmaceutical preparation which contains a compound of the formula I and/or one of its physiologically acceptable salts.

5. Use of a compound of the formula I and/or one of its physiologically acceptable salts for the production of medicaments.

## Claims (Claims for the following Contracting State(s): ES)

1. Process for the preparation of 3-(3-indolyl)quinuclidine derivatives of the formula I
Ind - R I
in which
Ind is a 3-indolyl group which is unsubstituted or monosubstituted to tetrasubstituted by 1-4-C-alkyl, trifluoromethyl, 1-4-C-alkoxy, 1-4-C-alkylthio, fluorine, chlorine, hydroxyl, hydroxymethyl, 6-10-C-aryloxy, 7-11-C-aralkyloxy, 1-5-C-acyloxy, 6-10-C-aroyloxy, 1-4-C-alkylsulfonyloxy, 6-10-C-arylsulfonyloxy, carboxyl, 1-4-C-alkoxycarbonyl and/or methylenedioxy and
R is 3-quinuclidinyl or 2,3-dehydro-3-quinuclidinyl,
and of their salts, characterised in that compounds of the formula II
Ind-H II
are reacted with 3-quinuclidinone or one of its salts to give the 2,3-dehydro-3-quinuclidinyl compound (I,R = 2,3-dehydro-3-quinuclidinyl) and this is reduced, if desired, to the 3-quinuclidinyl compound (I,R = 3-quinuclidinyl), and/or a compound which otherwise corresponds to the formula I, but contains a removable protecting group instead of one or more H atoms, is converted into a compound of the formula I by removing this protecting group and/or a radical Ind in a compound of the formula I is converted into another radical Ind and/or a base of the formula I is converted into one of its salts by treating with an acid.

2. Process for the production of a pharmaceutical preparation, characterised in that a compound of the formula I and/or one of its physiologically acceptable salts is brought into a suitable administration form together with at least one solid, liquid or semi-liquid excipient or auxiliary.

## Revendications (Revendications pour l'(les) Etat(s) contractant(s) suivant(s): AT, BE, CH, LI, DE, DK, FR, GB, GR, IT, LU, NL, SE)

1. 3-(3-indolyl)-quinuclidines de formule I
Ind-R (I)
dans laquelle
· Ind représente un groupe 3-indolyle non substitué ou portant un à quatre substituants choisis parmi les groupes alkyle en C₁-C₄, trifluorométhyle, alcoxy en C₁-C₄, alkylthio en C₁-C₄, le fluor, le chlore, les groupes hydroxy, hydroxyméthyle, aryloxy en C₆-C₁₀, aralkyloxy en C₇-C₁₁, acyloxy en C₁-C₅, aroyloxy en C₆-C₁₀, alkylsulfonyloxy en C₁-C₄, arylsulfonyloxy en C₆-C₁₀, carboxy, (alcoxy en C₁-C₄)-carbonyle et/ou méthylène-dioxy et
· R représente un groupe 3-quinuclidinyle ou 2,3-déshydro-3-quinuclidinyle et leurs sels.

2. Procédé de préparation des composés de formule I et de leurs sels, caractérisé en ce que l'on fait réagir des composés de formule II
Ind-H II
dans laquelle Ind a les significations indiquées dans la revendication 1, avec la 3-quinuclidinone ou l'un de ses sels, ce qui donne le dérivé 2,3-déshydro-3-quinuclidinylique (I, R = 2,3-déshydro-3-quinuclidinyle) qu'on réduit si on le désire en le dérivé 3-quinuclidinylique (I, R = 3-quinuclidinyle) et/ou on convertit un composé répondant par ailleurs à la formule I mais portant un groupe protecteur éliminable à la place d'un ou plusieurs atomes d'hydrogène, par scission de ces groupes protecteurs, en un composé de formule I, et/ou, dans un composé de formule I, on convertit un groupe Ind en un autre Ind, et/ou on convertit une base de formule I en l'un de ses sels en la traitant par un acide.

3. Procédé de préparation d'une composition pharmaceutique, caractérisé en ce que l'on met sous une forme d'administration appropriée un composé de formule I et/ou l'un de ses sels acceptables pour l'usage pharmaceutique avec au moins un véhicule ou produit auxiliaire solide, liquide ou semi-liquide.

4. Composition pharmaceutique, caractérisée en ce qu'elle contient un composé de formule I et/ou l'un de ses sels acceptables pour l'usage pharmaceutique.

5. Utilisation d'un composé de formule I et/ou de l'un de ses sels acceptables pour l'usage pharmaceutique pour la préparation de médicaments.

## Revendications (Revendications pour l'(les) Etat(s) contractant(s) suivant(s): ES)

1. Procédé de préparation de dérivés de la 3-(3-indolyl)-quinuclidine de formule I
Ind - R I
dans laquelle
· Ind représente un groupe 3-indolyle non substitué ou portant un à quatre substituants choisis parmi les groupes alkyle en C₁-C₄, trifluorométhyle, alcoxy en C₁-C₄, alkylthio en C₁-C₄, le fluor, le chlore, les groupes hydroxy, hydroxyméthyle, aryloxy en C₆-C₁₀, aralkyloxy en C₇-C₁₁, acyloxy en C₁-C₅, aroyloxy en C₆-C₁₀, alkylsulfonyloxy en C₁-C₄, arylsulfonyloxy en C₆-C₁₀, carboxy (alcoxy en C₁-C₄)-carbonyle et/ou méthylène-dioxy et
· R représente un groupe 3-quinuclidinyle ou 2,3-déshydro-3-quinuclidinyle
· et de leurs sels,
caractérisé en ce que l'on fait réagir des composés de formule II
Ind-H II
avec la 3-quinuclidinone ou l'un de ses sels, ce qui donne le dérivé 2,3-déshydro-3-quinuclidinylique (I, R = 2,3-déshydro-3-quinuclidinyle) qu'on réduit si on le désire en le dérivé 3-quinuclidinylique (I, R = 3-quinuclidinyle) et/ou on convertit un composé répondant par ailleurs à la formule I mais portant un groupe protecteur éliminable à la place d'un ou plusieurs atomes d'hydrogène, par scission de ces groupes protecteurs, en un composé de formule I, et/ou, dans un composé de formule I, on convertit un groupe Ind en un autre groupe Ind et/ou on convertit une base de formule I en l'un de ses sels par traitement à l'aide d'un acide.

2. Procédé de préparation d'une composition pharmaceutique caractérisé en ce que l'on met sous une forme d'administration appropriée un composé de formule I et/ou l'un de ses sels acceptables pour l'usage pharmaceutique avec au moins un véhicule ou produit auxiliaire solide, liquide ou semi-liquide.
